Europäisches Patentamt

European Patent Office

Office européen des brevets

(11) Publication number: 0 307 173
A1

# EUROPEAN PATENT APPLICATION

(21) Application number: 88308247.1

(22) Date of filing: 07.09.88

(51) Int. Cl.⁴: A61L 2/26 , A61J 1/00 , B65D 75/30 , B65D 33/00

(30) Priority: 11.09.87 FI 873939

(43) Date of publication of application:
15.03.89 Bulletin 89/11

(84) Designated Contracting States:
AT BE CH DE ES FR GB IT LI NL SE

(71) Applicant: A. AHLSTROM CORPORATION

SF-29600 Noormarkku(FI)

(72) Inventor: Juselius, Jukka
Lohiluoma as. 535
SF-27500 Kauttua(FI)
Inventor: Saarinen, Hannu
Sofianlehdonkatu 7 D 37
SF-00610 Helsinki(FI)

(74) Representative: Gilmour, David Cedric
Franklyn et al
POTTS, KERR & CO. 15 Hamilton Square
Birkenhead Merseyside L41 6BR(GB)

(54) Disposable sterilization package.

(57) The present invention relates to a disposable sterilization package especially for packing hospitals utensils. Known packages are formed partly of porous paper, which especially after vapor sterilization is weakened to such an extent that it may break up for minor reasons. Additionally, when opening the package, fibers from the paper layer may be detached onto the surface of the object put into the package, thus risking contamination of the object.

The package in accordance with the invention comprises partly of a plastics material and partly of a non-woven or other material, whereby the non-woven or other material is such as to permit the breathing of the package required in the sterilization, whilst being such as to prevent bacteria or other contaminants from entering the package after the sterilization.

FIG. 5

# DISPOSABLE STERILIZATION PACKAGE

The present invention relates to a disposable sterilization package for different kinds of hospital utensils and like requiring sterilization, in other words instruments, needles, syringes, catheters, cotton wool, gloves and like objects.

The sterilization packages available at present typically consist of special sterilizable paper and plastics foil or laminate. The paper is a water strength special paper (such as Sterirex, Kauttua Paper Mill, A. Ahlstrom Corporation) having a certain pore size. The plastics laminate is a polyester/polypropylene-laminate (e.g. PETP/PP), in which the polypropene is seamed against the paper thus closing the package.

Sterilization is carried out either as vapor sterilization in an autoclave or as gas sterilization, in which methods both the vapor and the gas penetrate a porous paper sterilizing the packed product. The paper does not however, let any bacteria in the package after the sterilization. The determination of the permeability of the paper is carried out by selecting the requisite pore size of the paper.

The mechanical strength of the paper in the described prior art package is poor, especially after the vapor sterilization, and as a consequence the amount of damaged packages is high. Additionally, when the package is opened pulp fibers from the package are easily detached, which fibers, when falling on the packed object, contaminate the object.

When using a package in accordance with the present invention, the mentioned drawbacks can be eliminated or minimized. The packages according to the invention can, however, be manufactured by conventional, traditional packing machines and manufacture lines, whereby the seamings can be made by heat sealing.

The package according to the invention is characterized in that at least part of the package is of porous non-woven or other material which is such as to permit sterilization but prevent bacteria or like contamination from penetrating the package after sterilization and which material is such that fibers are not or at least not readily loosened therefrom when opening the package.

The package in accordance with the invention is described more in detail below, by way of example, with reference to the accompanying drawings, in which:

Fig. 1 is a schematic cross-sectional view of a package according to the prior art;

Fig. 2 is a shematic cross-sectional view of another package according to the prior art;

Fig. 3 is a schematic cross-sectional view of a package forming a first embodiment of the invention;

Fig. 4 is a schematic cross-sectional view of a package forming a second embodiment of the invention;

Fig. 5 is a schematic cross-sectional view of a package forming a third embodiment of the invention; and

Fig. 6 is a schematic cross-sectional view of a package forming a fourth embodiment of the invention.

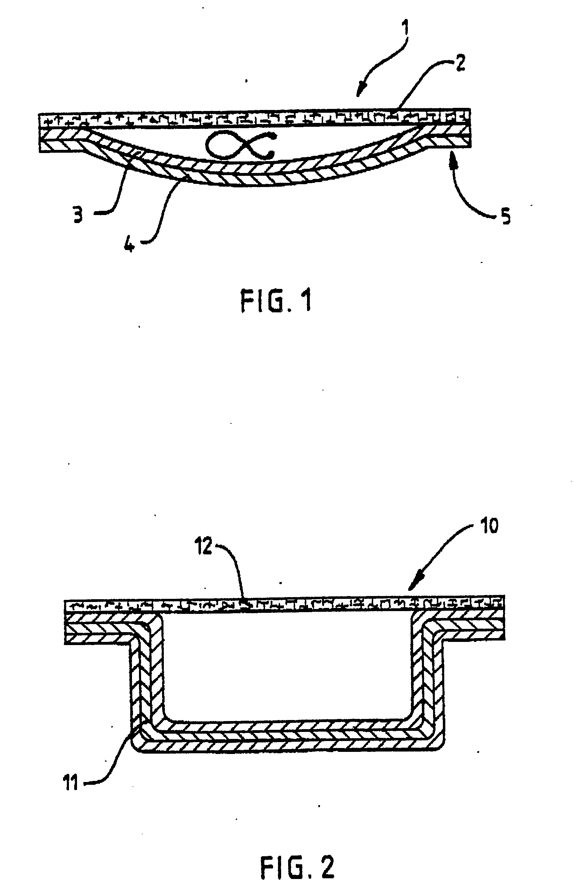

Fig. 1 shows a typical bag-shaped package 1, which consists of a porous paper element 2 and a two-layer plastics laminate. The plastics laminate includes a polypropene layer 3 and a polyesther layer 4, which are seamed on the edges 5 together with the paper 2.

Fig. 2 illustrates a track-shaped package 10 formed by an automatic track machine, which package typically comprises a lower track 11 of plastics laminate and an upper track 12 of porous paper. Known laminates used for the lower track 11 are, for example, poly- ethylene/adhesive polymer/polyamide-6/adhesive polymer/polyethylene.

As has already been mentioned above, vapor sterilization especially weakens the strength of the paper elements of the package, because some of the vapor remains in the paper layer and weakens the bonds between the fibers, and as a result of which even a considerably small stress can cause the breaking of the paper layer and exposure of the packed objects to bacteria and other impurities. As a consequence, a large number of the packed products must be returned to be repacked and resterilized.

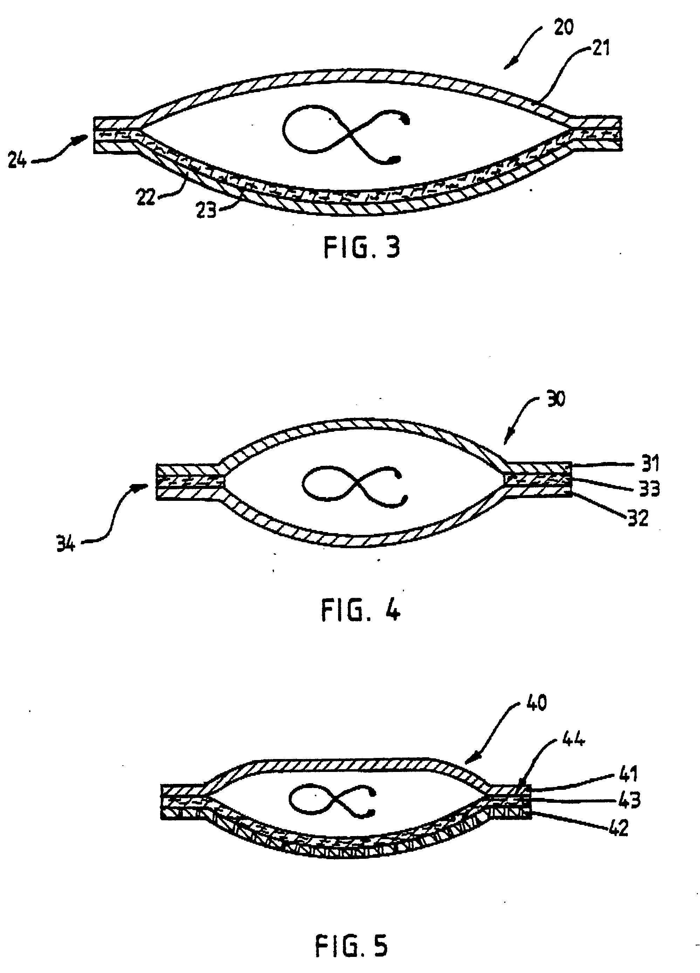

Fig. 3 illustrates a package 20 in accordance with a first preferred embodiment of the invention comprising a plastics material foil 21 and a plastics material laminate 22/23. In the seaming of the package there is utilized a porous, web-like or gauze-like fiber cloth 23 being part of the laminate 22/23, or alternatively, a non-woven cloth or like material. The non-woven cloth can include polypropene, polyesther polyamide or polyethylene. The non-woven cloth operates in the region of seam 24 as a filter and is such as to permit sterilization, but not to allow bacteria inside the package. The package can be manufactured as a bag or, alternatively, automatic track machines may be used to form a track-shaped package. In the arrangement according to the invention the seam between the non-woven cloth and the plastics material, which seam is tight and easy to open without loosening fibers from the package to the packed product, is effected by means of known methods, such as heat sealing.

When vapor sterilization is used, polypropene is advantageously used both as the seaming layers and as the raw material of the non-woven cloth. By using this kind of plastics construction, the package becomes a several times stronger and is transparent package, which can be cleanly opened.

According to the second embodiment illustrated in Fig. 4, a non-woven cloth 33 is located in a package 30 only in the seaming area 34, whereby both walls 31 and 32 of the package are plastics material foils or plastics material laminates. By this method some saving can be gained in the packaging costs. However, the principle of operation of the package corresponds exactly to that of the package in Fig. 3, because the seam 34 is still "breathing", i.e. permits sterilization of the contents whilst preventing contamination.

Fig. 5 discloses as a third embodiment of the invention a package 40, which comprises on one side a plastics material foil or a plastics laminate 41 and, on the other side, a plastics/ non-woven laminate 42/43, whereby the plastics layer 42 is perforated. The plastics/non-woven laminate wall is highly permeable to sterilizing vapors but impermeable to bacteria and other contaminating microorganism. The formation of the package is carried out by bonding the non-woven layer 43 from the edges to the plastics laminate layer 41.

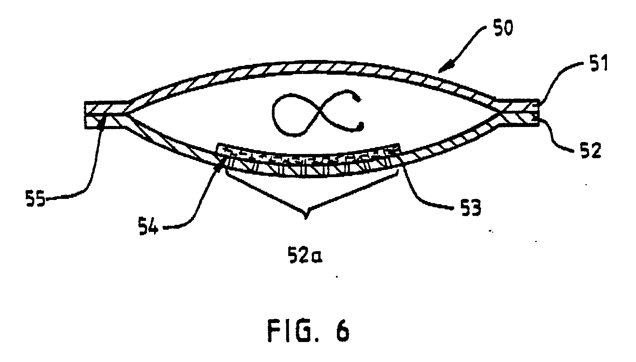

In the embodiment shown in Fig. 6 the package 50 comprises one impermeable wall 51, made of a plastics material foil or a plastics laminate, and one wall 52 made of a plastics foil or a plastics laminate, a portion or part 52a of which is perforated. The inner surface of the perforated part of the wall is covered by a non-woven cloth 53 which is secured to the wall by a continuous seal 54 around the perforated area. The walls 51 and 52 are bonded together along the outer edges by heat sealing at 55 to close the package. The perforated part of the wall covered by the non-woven cloth provides a breathing area and a bacteria barrier to permit sterilization whilst preventing entry of bacteria.

Yet a further embodiment is to be mentioned, namely, a package in which one side or a substantial part of the package is totally formed by a non-woven cloth, wherein the non-woven layer is considerably thicker than that used in the mentioned plastics laminate, because the mechanical strength of the non-woven cloth would not otherwise be sufficient. By using this kind of thicker layer the price of the package may be a little higher, but in some cases the use of this kind of package is justified.

The non-woven cloth used in the invention typically has a thickness of 5 - 50 g/m². However, it must be born in mind, as it has been noted above, that the non-woven cloth is not the only possible porous material to be used in the packages as a breathing surface, but also other porous materials, such as web-like, breathing products made by melt blow method, are possible.

As it is noted from the above description, a new type of disposable sterilization package has been developed which is more secure than those of the prior art and more economic. Only a few preferred embodiments of the package of the invention are described above, which are by no means intended to restrict the invention from what is defined in the accompanying claims, which define the scope of the invention.

## Claims

1. A disposable sterilization package, which at least partially includes a porous surface, characterized in that at least part of the package (20, 30, 40, 50) is of porous non-woven or other material (23, 33, 43, 53), which is such as to permit sterilization to be effected but prevent bacteria and like contaminants from entering the package after the sterilization; said material also being such that fibers are not or not readily detached therefrom when opening the package.

2. A package according to claim 1, characterized in that the seam (24, 34, 44) of the package (20, 30, 40) is of or includes a layer of porous material.

3. A package according to claim 1, characterized in that at least part of the walls of the package (40, 50) is formed of laminate, which is a perforated plastics foil or a plastics laminate material (42, 52), on at least one side of which there is said porous, non-woven or other material layer (43, 53).

4. A package according to claim 3, characterized in that said non-woven or other material layer (43, 53) covers the inner surface of the perforated part (42, 52a) of the wall.

FIG. 1

FIG. 2

FIG. 3

FIG. 4

FIG. 5

FIG. 6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 644 586 (L.W. PADGETT)<br>* Column 3, lines 53-67; column 4, lines 6,7,44-50 * | 1,2 | A 61 L 2/26<br>A 61 J 1/00<br>B 65 D 75/30<br>B 65 D 33/00 |
| Y | | 4 | |
| X | US-A-4 660 721 (L.G. MYKLEBY)<br>* Column 6, lines 25-36 * | 1,2 | |
| X | DE-A-2 952 751 (K.H. SENGEWALD)<br>* Page 11, line 11 * | 1,2 | |
| X | US-A-4 367 816 (K.R. WILKES)<br>* Column 1, lines 52-59; column 4, lines 58-62; claim 15 * | 1,3 | |
| X | GB-A-1 436 489 (S.J. SCHUSTER)<br>* Page 3, lines 39-51 * | 1 | |
| X | US-A-4 466 552 (G.A.M. BUTTERWORTH)<br>* Column 6, lines 12-27 * | 1 | |
| Y | US-A-3 229 813 (G.A. CROWE)<br>* Column 4, lines 1-12; figure 2 * | 4 | **TECHNICAL FIELDS SEARCHED (Int. Cl.4)**<br><br>A 61 L 2/26 |
| A | EP-A-0 089 659 (SURGICOT)<br>* Page 4, lines 28-30 * | 1,2 | |
| A | WO-A-8 001 062 (D. LENNARD)<br>* Claim 6 * | 1 | |

The present search report has been drawn up for all claims

| Place of search | Date of completion of the search | Examiner |
|---|---|---|
| THE HAGUE | 09-11-1988 | PELTRE CHR. |